# EUROPEAN PATENT APPLICATION

(11) **EP 2 011 872 A1**
(43) Date of publication of application: **07.01.2009**
(21) Application number: 08012041.3
(22) Date of filing: 03.07.2008
(51) Int. Cl.: C12N 15/10, C12Q 1/68, G01N 1/40

(54) **Instrument and method for nucleic acid isolation**

(30) Priority: 04.07.2007 JP 2007176325
(71) Applicant: Hitachi High-Technologies Corporation, Tokyo 105-8717 (JP)
(72) Inventor: Yamashita, Yoshihiro, Hitachinaka-shi Ibaraki 312-8504 (JP); Yamamoto, Shuhei, Hitachinaka-shi Ibaraki 312-8504 (JP); Shoji, Yoshiyuki, Hitachinaka-shi Ibaraki 312-8504 (JP); Sakurai, Toshinari, Hitachinaka-shi Ibaraki 312-8504 (JP); Umetsu, Hiroshi, Hitachinaka-shi Ibaraki 312-8504 (JP)
(74) Representative: Strehl Schübel-Hopf & Partner

(57) **Abstract**

According to this invention, an instrument and a method for nucleic acid isolation is realized, whereby it is possible to prevent, for example, adherence of a discharged solution upon dispersion of such solution to the vicinity of a nucleic acid isolation column outlet.

A light source unit and a light-receiving unit that receives totally reflected light of the photosensor 41 for detecting the fluid level are placed on a site on the side surface of the nucleic acid isolation column 35, where the photosensor can detect the fluid level corresponding to a remaining liquid volume of approximately 10 ul. In addition, the light source unit of the photosensor 41 is positioned in a manner such that incident light is totally reflected from the interface between the nucleic acid isolation column 35 and air in the nucleic acid isolation column 35 and light is allowed to be incident in the solution when reaching the interface between the nucleic acid isolation column 35 and the solution in the nucleic acid isolation column 35. The light-receiving unit of the photosensor 41 is positioned in a manner such that it receives the totally reflected light and is fixed on the upper part of the nucleic-acid-adsorptive solid phase 24. The fluid level detection sensor is in communication with a syringe-driving motor 34 via a control unit 40 such that a syringe 31 is driven upward when the fluid level reaches a predetermined level, provided that a certain volume of the liquid remains in the nucleic acid isolation column 35. Accordingly, dispersion of the dispersed liquid is prevented.

## Description

### BACKGROUND OF THE INVENTION

### Field of the Invention

The present invention relates to a method and an instrument for nucleic acid isolation, wherein nucleic acids are isolated from a sample containing nucleic acids.

### Background Art

Analysis of nucleic acids, which are substances that carry genetic information, is being carried out in a wide variety of fields, such as academic research and medicine. Nucleic acid analysis methods often involve nucleic acid amplification techniques, typically polymerase chain reaction (PCR). Herein, "PCR" refers to a method for amplifying nucleic acids in a nucleotide-sequence-specific manner, whereby a gene of interest can be detected or quantified in minute amounts.

Upon nucleic acid analysis involving PCR or the like, nucleic acids isolated from a biological sample or the like are used. Upon nucleic acid isolation, it is necessary to remove the influence of contaminants, including inhibitors of an analysis reaction, such as nucleic acids derived from the external environment that cause false-positive analytical results.

In one conventional method for nucleic acid isolation from a biological sample, an operation such as ethanol precipitation or the like is carried out using poisonous material such as phenol or chloroform. In recent years, it has been common to use a method utilizing a property of nucleic acids whereby nucleic acids adsorb to a silica-containing nucleic-acid-adsorptive solid phase or the like in the presence of a chaotropic agent or a method utilizing a property of nucleic acids whereby nucleic acids adsorb to organic macromolecules such as acetylcellulose in the presence of a chaotropic agent and an aqueous organic solvent as described in JP Patent Publication (Kokai) No. 2005-204578 A.

The method described in JP Patent Publication (Kokai) No. 2005-204578 A is a method wherein a solution containing nucleic acids is introduced into an isolation cartridge comprising, as nucleic-acid-adsorptive members, organic macromolecules such as acetylcellulose and the solution is discharged through the nucleic-acid-adsorptive members by pressurization, such that nucleic acids are allowed to adsorb to the nucleic-acid-adsorptive members.

In such case, a pressure change in a nucleic acid isolation column immediately after the discharge of the entire volume of a solution is analyzed, a pressure release valve is operated, and then the pressurized air in the nucleic acid isolation column is released. Accordingly, dispersion of the discharged solution in a mist form caused by the remaining pressurized air is prevented such that risks of causing contamination are reduced.

### SUMMARY OF THE INVENTION

However, as in the case of the method described in the aforementioned JP Patent Publication (Kokai) No. 2005-204578 A, when a pressure change in a nucleic acid isolation column immediately after the discharge of the entire volume of a solution is detected, it is impossible to completely prevent dispersion of the discharged solution in a mist or droplet form, which occurs immediately after discharge of the entire volume of the solution.

In particular, in the case of a highly viscous solution containing a biological sample, a surfactant, and the like, it is necessary to apply high pressure in order to allow such a solution to pass, and thus the residual pressure after discharge of the entire volume of the solution becomes high. In addition, in such case, bubbles are sometimes formed when the entire volume of the solution is discharged. During formation of such bubbles, it is difficult to detect a pressure change in a nucleic acid isolation column.

Thus, contamination might be caused as a result of dispersion of a discharged solution in a mist or droplet form following disruption of bubbles caused at the residual pressure or as a result of adherence of bubbles (formed at the residual pressure) to the vicinity of a nucleic acid isolation column outlet.

It is an objective of the present invention to realize an instrument and a method for nucleic acid isolation, whereby it is possible to prevent, for example, adherence of a discharged solution as a result of dispersion of such solution to the vicinity of the nucleic acid isolation column outlet.

In order to solve the above problems, the constitution of the present invention is described as follows.

In a method for nucleic acid isolation, wherein nucleic acids of interest are isolated from a liquid sample containing nucleic acids, such method comprises the steps of:
allowing nucleic acids to adsorb to the nucleic-acid-adsorptive solid phase by aspirating the liquid sample containing nucleic acids into the nucleic acid isolation column and discharging the liquid sample containing nucleic acids from the nucleic acid isolation column, provided that a certain volume of the liquid sample containing nucleic acids is allowed to remain in the nucleic acid isolation column;
removing nonspecific adsorbing substances from the nucleic-acid-adsorptive solid phase by aspirating a washing reagent into the nucleic acid isolation column and discharging the washing reagent and the nonspecific adsorbing substances from the nucleic acid isolation column, provided that a certain volume of the washing reagent is allowed to remain in the nucleic acid isolation column; and
eluting nucleic acids of interest from the nucleic-acid-adsorptive solid phase by aspirating an elution reagent into the nucleic acid isolation column and discharging the elution reagent and nucleic acids of interest from the nucleic acid isolation column, provided that a certain volume of the elution reagent is allowed to remain in the nucleic acid isolation column.

In addition, regarding an instrument for nucleic acid isolation, in which nucleic acids of interest are isolated from a liquid sample containing nucleic acids, such instrument comprises:
a nucleic acid isolation column having a nucleic-acid-adsorptive solid phase;
an aspiration and discharge unit for aspirating a solution into the nucleic acid isolation column and discharging the solution therefrom;
a remaining volume detection unit for detecting whether or not a certain volume of the solution remains in the nucleic acid isolation column; and
a control unit for controlling the operation of the aspiration and discharge unit and terminating a discharge operation of the aspiration and discharge unit based on a detection signal from the remaining volume detection unit.

Further, such control unit controls an operation of the aspiration and discharge unit so as to cause elution of nucleic acids of interest from the nucleic-acid-adsorptive solid phase by the steps of:
aspirating the liquid sample containing nucleic acids into the nucleic acid isolation column and discharging the liquid sample containing nucleic acids from the nucleic acid isolation column, provided that a certain volume of the liquid sample containing nucleic acids is allowed to remain in the nucleic acid isolation column;
aspirating a washing reagent into the nucleic acid isolation column and discharging the washing reagent and nonspecific adsorbing substances from the nucleic acid isolation column, provided that a certain volume of the washing reagent is allowed to remain in the nucleic acid isolation column; and
aspirating an elution reagent into the nucleic acid isolation column and discharging the elution reagent and nucleic acids of interest from the nucleic acid isolation column, provided that a certain volume of the elution reagent is allowed to remain in the nucleic acid isolation column.

### Effects of the Invention

It is possible to realize a method and an instrument for nucleic acid isolation, whereby it is possible to prevent dispersion of a discharged solution in a mist or droplet form, such dispersion occurring immediately after discharge of the entire volume of the solution, or to prevent adherence of a discharged solution to the vicinity of a nucleic acid isolation column outlet as a result of disruption of bubbles.

### BRIEF DESCRIPTION OF THE DRAWINGS

Fig. 1 is a general operational flowchart of a method for nucleic acid isolation in one embodiment of the present invention.
Fig. 2 is an explanatory drawing of a nucleic acid isolation column comprising a nucleic-acid-adsorptive solid phase used in one embodiment of the present invention.
Fig. 3 is an explanatory drawing of a pressurization mechanism of a nucleic acid isolation column used in one embodiment of the present invention.
Fig. 4 shows one example of a fluid level detection unit of a nucleic acid isolation column used in one embodiment of the present invention.
Fig. 5 shows another example of a fluid level detection unit of a nucleic acid isolation column used in one embodiment of the present invention.

### DESCRIPTION OF THE PREFERRED EMBODIMENTS

Hereinafter, embodiments of the present invention are described by referring to the drawings.

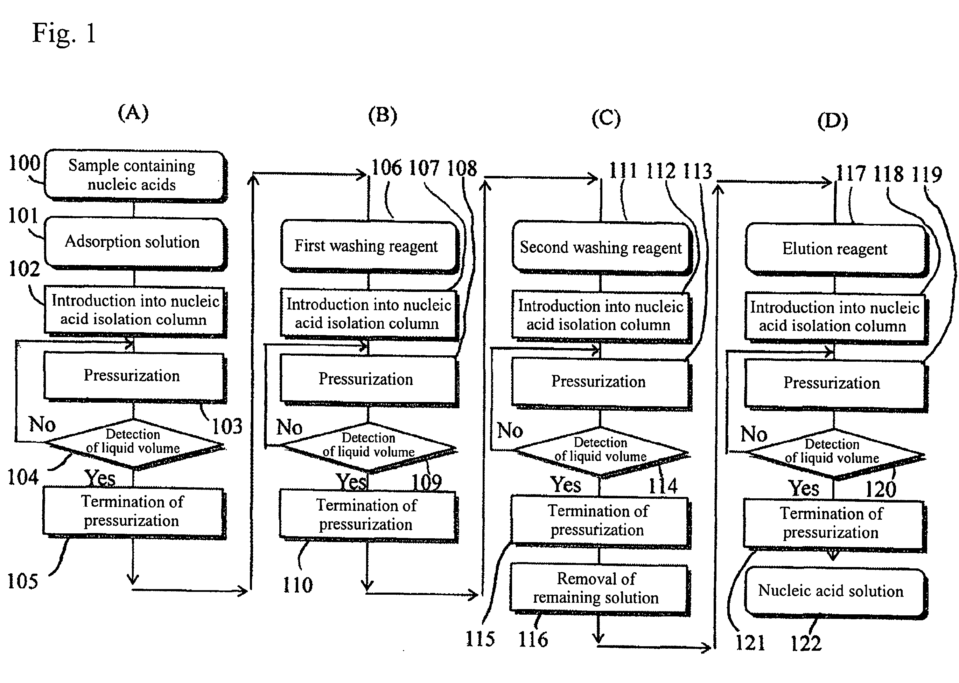

Fig. 1 is a general operational flowchart of a method for nucleic acid isolation in one embodiment of the present invention. The method for nucleic acid isolation comprises a nucleic acid adsorption step (A), a first washing step (B), a second washing step (C), and a nucleic acid elution step (D).

The nucleic acid adsorption process (A) comprises introducing a liquid mixture obtained by adding an adsorption reagent to a sample containing nucleic acids into a nucleic acid isolation column, pressurizing the inside of the nucleic acid isolation column such that the liquid mixture is allowed to pass through a nucleic-acid-adsorptive solid phase so as to be discharged, detecting that the remaining liquid volume in the nucleic acid isolation column has become a predetermined volume, and releasing pressure (corresponding to steps 100 to 105).

The first washing process (B) comprises introducing a first washing reagent into a nucleic acid isolation column, pressurizing the inside of the nucleic acid isolation column such that the washing reagent is allowed to pass through the nucleic-acid-adsorptive solid phase so as to be discharged, detecting that the remaining liquid volume in the nucleic acid isolation column has become a predetermined volume, and releasing pressure (corresponding to steps 106 to 110).

The second washing process (C) comprises introducing a second washing reagent into a nucleic acid isolation column, pressurizing the inside of the nucleic acid isolation column such that the washing reagent is allowed to pass through the nucleic-acid-adsorptive solid phase so as to be discharged, detecting that the remaining liquid volume in the nucleic acid isolation column has become a predetermined volume, releasing pressure, and removing the remaining solution (corresponding to steps 111 to 116).

The first washing process (B) and the second washing process (C) each correspond to a step of allowing nucleic acids of interest to remain in a nucleic-acid-adsorptive solid phase and removing the other nucleic acids.

The nucleic acid elution process (D) comprises introducing an elution reagent into a nucleic acid isolation column, pressurizing the inside of the nucleic acid isolation column such that the elution reagent is allowed to pass through the nucleic-acid-adsorptive solid phase so as to be discharged, detecting that the remaining liquid volume in the nucleic acid isolation column has become a predetermined volume, releasing pressure, and discharging nucleic acids of interest in the nucleic-acid-adsorptive solid phase so as to obtain a nucleic acid solution (corresponding to steps 117 to 122).

Examples of samples containing nucleic acids that can be used include body fluids such as whole blood, plasma, serum, urine, saliva, sputum, or seminal fluid, tissues or cells of plants and animals, microorganisms, bacteria, viruses, and suspensions containing any thereof or lysates of any thereof.

Also, examples of adsorption reagents that can be used include a variety of chaotropic salts, which allow nucleic acids in a sample containing nucleic acids to be released and promote adsorption of nucleic acids to a nucleic-acid-adsorptive solid phase. Examples of such chaotropic salt that can be used include guanidine thiocyanate, guanidine hydrochloride, sodium iodide, potassium iodide, and sodium perchlorate.

In addition, in order to promote release of nucleic acids, a surfactant, a protease, and the like can be used.

Preferred examples of surfactants that can be used include nonionic surfactants such as polyoxyethylene (10) octylphenyl ether and polyoxyethylene (20) sorbitan monolaurate.

In addition, in order to deactivate a nucleotidase contained in a sample containing nucleic acids or derived from an external environment, reductants such as 2-mercaptoethanol and dithiothreitol and metal chelating agents such as ethylenediamine tetraacetate can be used.

Further, in order to promote release of nucleic acids, heating, agitation, homogenization, and ultrasonication treatments can be used in combination. Furthermore, in order to promote adsorption of nucleic acids to a nucleic-acid-adsorptive solid phase, an organic solvent can be used. Preferred examples of organic solvents that can be used include aqueous organic solvents such as methanol, ethanol, isopropanol, butanol, acetone, diethylene glycoldimethyl ether, and ethyl lactate.

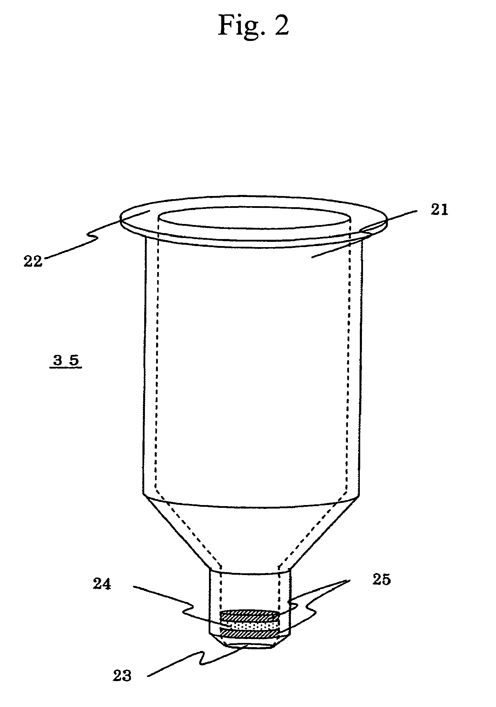

Fig. 2 is an explanatory drawing of a nucleic acid isolation column 35 comprising a nucleic-acid-adsorptive solid phase that is used in one embodiment of the present invention.

In fig. 2, the nucleic acid isolation column 35 comprises an opening 21 through which a solution is introduced, a connecting portion 22 to which a pressurization mechanism is connected, and a tube 26 having an opening 23 through which the solution is discharged. A nucleic-acid-adsorptive solid phase 24 is provided inside the tube 26, such solid phase being fixed with a supporting member 25.

An example of a nucleic-acid-adsorptive solid phase 24 that can be used is a solid phase comprising glass, silica, diatom earth, acetylcellulose, or the like. Such solid phase has a fine and tightly knitted filter-like structure having a plurality of consecutive liquid passage pores through which a solution can pass, such that a solution passes through the solid phase with sufficient efficiency of contact between the solution and the solid phase.

A supporting member 25 that can be used is a member capable of maintaining the shape and installation position of a nucleic-acid-adsorptive solid phase 24 and having a plurality of consecutive liquid passage pores through which a solution can pass. Examples thereof include sintered polypropylene particles.

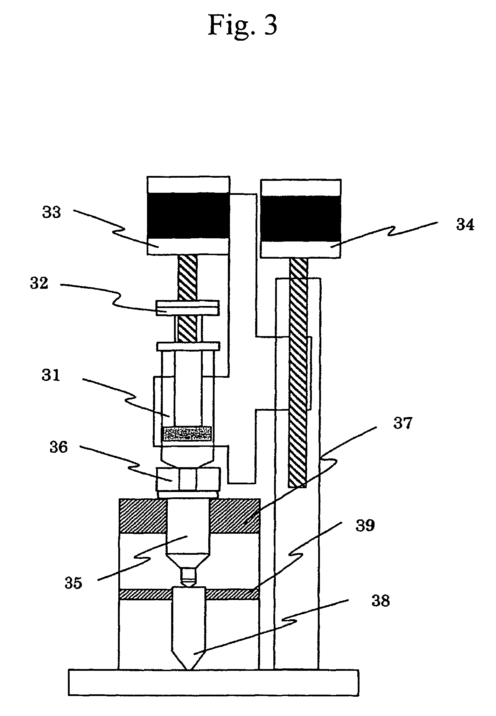

Fig. 3 is an explanatory drawing of a pressurization mechanism whereby the inside of a nucleic acid isolation column 35 into which a solution has been introduced is pressurized such that the solution is allowed to pass through a nucleic-acid-adsorptive solid phase 24 so as to be discharged.

In fig. 3, the pressurization mechanism comprises: a syringe 31 that is used to pressurize the inside of a nucleic acid isolation column 35; a plunger 32; a plunger-driving motor 33 that drives the plunger 32 in the vertical direction; a syringe-driving motor 34 that drives the syringe 31 and the plunger 32 in the vertical direction; a sealing member 36 used between the syringe 31 and the nucleic acid isolation column 35; a nucleic acid isolation column-supporting stand 37; a waste solution reservoir 38; and a waste solution reservoir-supporting stand 39.

In the case of the above pressurization mechanism, the plunger 32 and the syringe 31 are moved upward and the nucleic acid isolation column 35 and the waste solution reservoir 38 are each placed on the relevant supporting stand. Then, a certain solution is introduced into the nucleic acid isolation column 35, the syringe 31 is moved downward, and the syringe 31 and the nucleic acid isolation column 35 are allowed to adhere to each other via the sealing member 36. Further, the plunger 32 is moved downward and the inside of the nucleic acid isolation column 35 is pressurized, such that the solution in the nucleic acid isolation column 35 is allowed to pass through inside the nucleic-acid-adsorptive solid phase 24 so as to be discharged into the waste solution reservoir 38.

The nucleic-acid-adsorptive solid phase 24 in the nucleic acid isolation column 35 has a fine and tightly knitted liquid passage pore structure. Thus, in a case of a highly viscous solution such as a liquid mixture of a sample and an adsorption solution, the liquid passage resistance becomes high. Therefore, in order to efficiently carry out liquid passage treatment, application of high pressure is necessary.

However, when the passage of a solution has been achieved by applying high pressure, the residual pressure becomes high immediately after the discharge of the entire volume of the solution. Accordingly, dispersion of the discharged solution in a mist or droplet form, dispersion of the discharged solution as a result of disruption of bubbles, adherence of bubbles to the vicinity of a nucleic acid isolation column outlet 23, or the like might occur.

Thus, it is necessary to avoid the risk of causing the above contamination by detecting a remaining liquid volume at a predetermined volume, releasing pressure immediately before discharging the entire volume of the solution, and terminating the discharge of the solution.

In the step of discharging a solution, a method for detecting that the remaining liquid volume in a nucleic acid isolation column 35 has become a predetermined volume may preferably be a method wherein detection is carried out in a solution-contactless manner. There are methods involving the use of different sensors such as a photosensor, a pressure sensor, an image sensor, and an ultrasonic sensor.

An example of a method using a photosensor is a method wherein a light source unit and a light-receiving unit of a photosensor are placed at a position at which the fluid level should be detected, such position being outside of the nucleic acid isolation column, and the fluid level is detected by measuring optical properties that change when the fluid surface passes the position at which the fluid level should be detected. Such optical properties that change as a result of the passage of the fluid surface are detected based on a difference between the refractive index of the solution and that of air.

For instance, in a case in which the fluid level is higher than such a fluid level sensor and the solution is contained in the nucleic acid isolation column 35, the refractive index of the nucleic acid isolation column 35 and that of the solution are small. Thus, incident light having a certain angle of incidence permeates the nucleic acid isolation column 35 and the solution. Meanwhile, in a case in which the fluid level is lower than the fluid level sensor and no solution is contained in the nucleic acid isolation column 35, the refractive index of the nucleic acid isolation column 35 and that of air are large. Thus, incident light having the above angle of incidence is totally reflected from the internal surface of the nucleic acid isolation column 35 or permeates the nucleic acid isolation column and the solution along a light beam path that differs from that of transmitted light in a case in which the solution is contained in the nucleic acid isolation column 35.

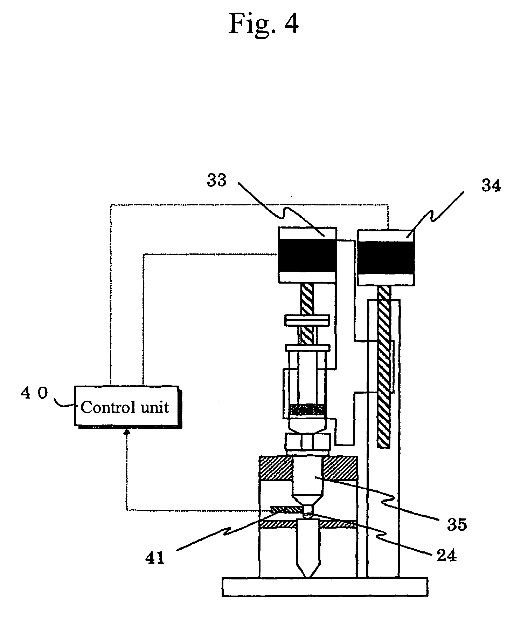

Fig. 4 is an explanatory drawing of a pressurization unit in a case in which the fluid level is detected using a photosensor 41. In fig. 4, in a pressurization unit using the photosensor 41, a light source unit and a light-receiving unit that receives totally reflected light of the photosensor 41 for detecting the fluid level are placed on a site on the side surface of the nucleic acid isolation column 35, where the photosensor can detect the fluid level corresponding to a remaining liquid volume of approximately 10 ul.

In addition, the light source unit of the photosensor 41 is positioned in a manner such that incident light is totally reflected from the interface between the nucleic acid isolation column 35 and air in the nucleic acid isolation column 35 and light is allowed to be incident in the solution when reaching the interface between the nucleic acid isolation column 35 and the solution in the nucleic acid isolation column 35. The light-receiving unit of the photosensor 41 is positioned in a manner such that it receives the above totally reflected light and is fixed on the upper part of the nucleic-acid-adsorptive solid phase 24. The fluid level detection sensor is in communication with a syringe-driving motor 34 via a control unit 40 such that a syringe 31 is driven upward when the fluid level reaches a predetermined level.

Next, the operational protocol of a pressurization unit using a photosensor 41 is explained.

First, a nucleic acid isolation column 35 and a waste liquid reservoir 38 are provided to a pressurization unit. Then, a liquid mixture of a sample (200 ul) and an adsorption solution (800 ul) is introduced into a nucleic acid isolation column and then the pressurization unit is activated. A syringe 31 is driven downward so as to adhere to a nucleic acid isolation column 35 via a sealing member 36. Thereafter, a plunger 32 is driven downward such that an air phase having an initial syringe volume of 5 ml is compressed to 1 ml (pressurization operation). The above operations correspond to operations in steps 100 to 103 in fig. 1.

Subsequently, it is detected whether or not the volume of the liquid mixture remaining in the nucleic acid isolation column 35 has become a predetermined volume (e.g., 10 ul). If not, the pressurization operation is continued (step 104). When the remaining volume has become 10 ul, the syringe 31 is driven upward so as to become disconnected from the nucleic acid isolation column 35, and the plunger 32 is driven upward (step 105).

Next, a first washing reagent (1200 ul) is introduced into the nucleic acid isolation column 35 (steps 106 and 107). The syringe 31 is driven downward so as to adhere to the nucleic acid isolation column 35 via the sealing member 36. The plunger 32 is driven downward such that an air phase having an initial syringe volume of 5 ml is compressed to 1 ml (step 108). Then, it is detected whether or not the volume of the liquid mixture remaining in the nucleic acid isolation column 35 has become a predetermined volume (e.g., 10 ul). If not, the pressurization operation is continued (step 109). When the remaining volume has become 10 ul, the syringe 31 is driven upward so as to become disconnected from the nucleic acid isolation column 35, and the plunger 32 is driven upward (step 110).

Next, a second washing reagent (1200 ul) is introduced into the nucleic acid isolation column 35 (steps 111 and 112). The syringe 31 is driven downward so as to adhere to the nucleic acid isolation column 35 via the sealing member 36. The plunger 32 is driven downward such that an air phase having an initial syringe volume of 5 ml is compressed to 1 ml (step 113). Then, it is detected whether or not the remaining volume of the washing reagent in the nucleic acid isolation column 35 has become a predetermined volume (e.g., 10 ul). If not, the pressurization operation is continued (step 114). When the remaining volume has become 10 ul, the syringe 31 is driven upward so as to become disconnected from the nucleic acid isolation column 35, and the plunger 32 is driven upward (step 115).

Steps 111 to 115 are carried out again. Then, the nucleic acid isolation column 35 is removed and heated at 65°C for 5 minutes. Accordingly, the remaining washing reagent is removed therefrom by volatilization (step 116).

Next, the waste liquid reservoir 38 is removed and discarded and then a purified liquid reservoir is positioned. Then, the nucleic acid isolation column 35 is positioned again. Thereafter, an elution reagent (110 ul) is introduced into the nucleic acid isolation column 35 (steps 117 and 118).

Then, the plunger 32 is driven downward such that an air phase (in the syringe 31) having an initial volume of 2 ml is compressed to 1 ml (step 119). Then, it is detected whether or not the remaining volume of the washing reagent in the nucleic acid isolation column 35 has become a predetermined volume (e.g., 10 ul). If not, the pressurization operation is continued (step 120). When the remaining volume has become 10 ul, the syringe 31 is driven upward so as to become disconnected from the nucleic acid isolation column 35, and the plunger 32 is driven upward (step 121). Thus, the elution reagent (approximately 100 ul) is obtained in the purified liquid reservoir (step 122).

In addition, regarding the aforementioned step of discharging a solution, there exists the following method for releasing pressure by detecting that the remaining liquid volume in the nucleic acid isolation column 35 has become a predetermined volume.

Specifically, there exists a method wherein a syringe 31 is moved upward by a control unit 40 that is in communication with a photosensor 41 and a motor 34 that drives the syringe in the vertical direction such that the syringe becomes disconnected from a nucleic acid isolation column 35, followed by release of the pressure in the nucleic acid isolation column 35.

Further, there exists another method wherein a plunger 32 is moved upward by a control unit 40 that is in communication with a photosensor 41 and a motor 33 that drives the plunger in the vertical direction, followed by release of the pressure in the nucleic acid isolation column 35.

Furthermore, there exists another example of such a method wherein a magnet valve is placed so as to be in communication with the inside of a syringe 31 and the magnet valve is opened. Alternatively, it is also possible to reduce the pressure in a stepwise manner until the remaining liquid volume in a nucleic acid isolation column 35 has become a predetermined volume and to completely release the pressure when the remaining liquid volume has become a predetermined volume.

There exists the following example of such method comprising reducing the pressure in a stepwise manner until the remaining liquid volume in a nucleic acid isolation column 35 has become a predetermined volume and completely releasing the pressure when the remaining liquid volume has become a predetermined volume: a method comprising detecting a decrease in a fluid level in a stepwise manner with at least one photosensor 41 that is placed at a site at which a fluid level at which the remaining liquid volume becomes a predetermined volume or a fluid level located above such level can be detected, moving a plunger 32 upward so as to reduce the pressure in a nucleic acid isolation column 35 in a stepwise manner, and completely releasing the pressure in the nucleic acid isolation column 35 when the remaining liquid volume has become a predetermined volume.

In addition, a first washing reagent is a solution capable of maintaining adsorption of nucleic acids to a nucleic-acid-adsorptive solid phase and removing nonspecific adsorbing substances such as proteins. Examples of a first washing reagent that can be used include a solution that can be used in an adsorption solution, such solution containing a chaotropic salt, a surfactant, a reductant, a metal chelating agent, an organic solvent, or the like.

Further, a second washing reagent is a solution capable of maintaining adsorption of nucleic acids to a nucleic-acid-adsorptive solid phase and removing nonspecific adsorbing substances, which were impossible to remove with a first washing reagent, and the remaining components of a first washing reagent to such an extent that the substances and the remaining components can be removed before an elution reagent is introduced in the subsequent step and thus they do not inhibit the subsequent analytical reaction. Examples of a second washing reagent that can be used include a solution containing, for example, a volatile organic solvent such as ethanol or acetone. As a method for removing a remaining portion of a second washing reagent, a method wherein a nucleic acid isolation column 35 is heated such that a remaining washing reagent is volatilized can be used.

In addition, an elution reagent is a solution that elutes nucleic acids from a nucleic-acid-adsorptive solid phase and does not contain components that inhibit the subsequent analytical reaction. Examples of such elution reagent that can be used include nucleotidase-free pure water and low-salt buffer. Further, in order to promote nucleic acid elution, an elution reagent can be heated.

Next, as a method for detecting whether the remaining liquid volume in a nucleic acid isolation column 35 has become a predetermined volume, a method using a pressure sensor is described.

The above method is a method for calculating the remaining liquid volume by measuring the pressure in a syringe 31 and that in an isolation column 35 with the use of a pressure sensor connected to the inside of the syringe 31. The pressure in the syringe 31 and that in the nucleic acid isolation column 35 upon pressurization are determined based on the initial position of and distance moved by a plunger 32 and the initial volume and remaining volume of a solution, respectively. For instance, in a case in which the initial position of a plunger 32 and the initial volume of a solution are stable, the remaining volume of the solution can be calculated based on the pressure in the nucleic acid isolation column 35 and the distance moved by the plunger in accordance with the driving force of a plunger-driving motor 33.

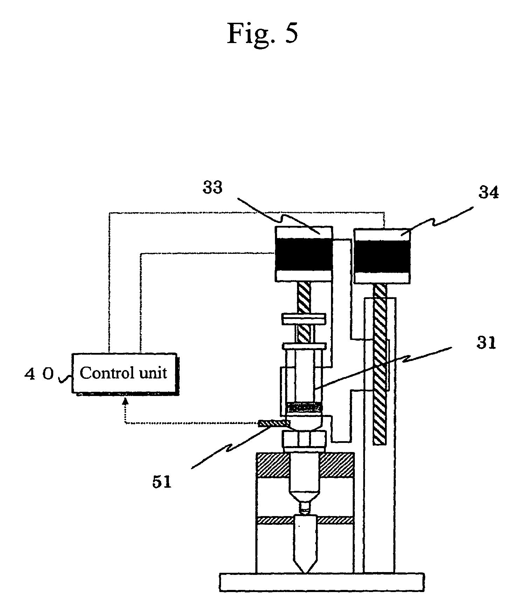

Fig. 5 is an explanatory drawing of a pressurization unit in a case in which a pressure sensor is used for detection of fluid level. In fig. 5, the pressurization unit used is a pressurization unit in which a pressure sensor 51 for detecting the remaining liquid volume is placed in a pressurization mechanism as shown in fig. 3 described above.

The pressure sensor 51 is connected to the side surface of the lower portion of the syringe via a communication path to the inside of the syringe 31. The remaining liquid volume is calculated based on the pressure value, the initial position of the plunger 32, and the distance moved by the plunger 32. In addition, the pressure sensor 51 is in communication with the plunger-driving motor 33 and the syringe-driving motor 34 via the control unit 40. Thus, when the pressure value reaches a level corresponding to a remaining liquid volume of approximately 10 ul, the syringe 31 is driven upward.

In addition, regarding the above step of discharging a solution, in addition to the method described in the case involving a photosensor, there exists the following method for releasing pressure by detecting that the remaining liquid volume in the nucleic acid isolation column 35 has become a predetermined volume.

Specifically, there exists the following method: a method comprising measuring the pressure in a nucleic acid isolation column 35 with a pressure sensor 51 so as to calculate the remaining liquid volume, reducing the pressure in the nucleic acid isolation column 35 in a stepwise manner by moving a plunger 32 upward until the remaining liquid volume in the nucleic acid isolation column 35 has become a predetermined volume, and completely releasing the pressure in the nucleic acid isolation column when the remaining liquid volume has become a predetermined volume; or a method comprising detecting the remaining liquid volume with an image sensor, reducing the pressure in the nucleic acid isolation column 35 in a stepwise manner by moving a plunger 32 upward until the remaining liquid volume in a nucleic acid isolation column 35 has become a predetermined volume, and completely releasing the pressure in the nucleic acid isolation column 35 when the remaining liquid volume has become a predetermined volume.

In addition, regarding the operational protocol of a pressurization unit using a pressurization sensor 51, a detailed explanation is omitted because the operations are similar to those in the case involving the use of a photosensor 41, except that the operational protocol differs from that of a pressurization unit using a photosensor 41 only in that termination of a pressurization operation is determined based on detection of pressure in the case involving the use of a pressurization sensor 51, while on the other hand, the same is determined based on detection of fluid level in the case involving the use of a photosensor 41.

In addition, regarding the subsequent analytical reaction for isolated nucleic acids obtained by treatments following step 122 shown in fig. 1, nucleic acid analysis involving the following can be applied: nucleic acid amplification, reverse transcription reactions, and nucleic acid elongation reactions, including PCR (Polymerase Chain Reaction), RT-PCR (Reverse Transcription-Polymerase Chain Reaction), LCR (Ligase Chain Reaction), SDA (Strand Displacement Amplification), NASBA (Nucleic Acid Sequence-Based Amplification), TMA (Transcription-Mediated Amplification), LAMP (Loop-mediated isothermal amplification), and ICAN (Isothermal and Chimeric primer-initiated Amplification of Nucleic acids); enzymatic reactions using restriction enzymes and the like; and electrophoresis.

Next, a sample, a reagent, an apparatus, and a method used in a verification experiment carried out in one embodiment of the present invention are described below.

The sample used was a false-positive specimen obtained by adding purified DNA of HBV-positive serum to HBV-negative serum (10³ copies/200 µl).

The reagent used was an adsorption reagent (6 M guanidine hydrochloride, 100 mM MES, 25 mM EDTA·2Na, and 20% (v/v) polyoxyethylene (10) octylphenyl ether).

The first washing reagent used contained 2.5 M guanidine thiocyanate, 100 mM MES, 25 mM EDTA·2Na, and 1% (v/v) polyoxyethylene (10) octylphenyl ether.

The second washing reagent used contained 10 mM Tris-HCl (pH 8.0) and 80% (v/v) EtOH.

The elution reagent used was 10 mM Tris-HCl (pH 8.0).

The nucleic acid isolation column used was a nucleic acid isolation column 35 as shown in fig. 2, such that the internal volume of the nucleic acid isolation column 35 was 2 ml, the material of the nucleic acid isolation column 35 was polypropylene, a nucleic-acid-adsorptive solid phase containing nucleic acids was a glass fiber filter paper having an average pore size of 0.7 µm, and a supporting member was a sintered body comprising polypropylene particles having an average particle retension size of approximately 100 µm.

PCR was used for a nucleic acid analysis method. PCR involving the use of HBV-derived DNA as template DNA was carried out under the following conditions in accordance with the existing report (J. Clin. Microbiol., 29(9), 1804-1811 (1991)).
Primer 1: 5'GCG GAT CCG AGT TAC TCT CGT TTT TGC 3'
Primer 2: 5'GCAAGC TTT CTA ACA ACA GTA GTT TCC GG 3'
Temperature conditions: 94°C (5 min) → 94°C (1 min) → 55°C (1 min) → 72°C (1 min) → 72°C (10 min) (Note that the cycle of 94°C (1 min) → 55°C (1 min) → 72°C (1 min) was repeated 30 times.)
LAMP: LAMP involving the use of HBV-derived DNA as template DNA was carried out under the following conditions in accordance with the existing report (Nucleic Acid Research, 28(12), e63 (2000)).
Primer FIP: 5'CCT GCT GCT ATG CCT CAT CTT CTT TGA CAA ACG GGC AAC
ATA CCT T 3'
Primer BIP: 5'GAT AAA ACG CCG CAG ACA CAT CCT TCC AAC CTC TTG TCC
TCC AA 3'
Primer F3: 5' GGT GGT TGA TGT TCC TGG A 3'
Primer B3: 5'CAAAAT TCG CAG TCC CCAAC 3'
Temperature conditions: 95°C (5 min) → On ice (3 min) → addition of Bst DNA polymerase → 65°C (1 h) → 80°C (10 min)
Confirmation of amplification products: Electrophoresis was carried out with the use of 0.8% agarose gel as a support. Staining with a fluorescent dye that adsorbs specifically to double-strand DNA was carried out, followed by confirmation under ultraviolet irradiation.
Experiments and results: The above sample was subjected to PCR using different reagents, a pressurization unit A (for detecting the liquid volume with the use of a photosensor), and an elution reagent (10 ul) obtained by carrying out DNA isolation in accordance with the operational protocol of the pressurization unit A. Likewise, LAMP was carried out using a pressurization unit B (for detecting the liquid volume with the use of a pressure sensor) and an elution reagent (10 ul) obtained by carrying out DNA isolation in accordance with the operational protocol of the pressurization unit B. Accordingly, each amplification product was confirmed.

As a result, in both cases, a good amplification product was obtained. Thus, it was verified that normal nucleic acid isolation was carried out and the resultant was applicable to the subsequent analysis reaction. In addition, in the step of discharging a solution by pressurization, it was confirmed that the pressure was released before the entire volume of the solution was discharged, and thus dispersion of the discharged solution in a mist or droplet form, dispersion of the discharged solution as a result of disruption of bubbles, or adherence of bubbles to the vicinity of a nucleic acid isolation column outlet (which happens immediately after the discharge of the entire volume of the solution) did not occur.

Thus, according to the present invention, it is possible to realize an instrument and a method for nucleic acid isolation, whereby it is possible to prevent, for example, adherence of a discharged solution upon dispersion of the solution to the vicinity of the nucleic acid isolation column outlet.

In addition, when the fluid level is detected using a photosensor, two photosensors are separately placed in the vertical direction. Thus, it is also possible to control the release of pressure by calculating the descending rate of the fluid level based on the time period between detection of the fluid level with the upper photosensor and detection of the fluid level with the lower photosensor and the distance between the photosensors upon detection and predicting the time at which a predetermined liquid volume is obtained. There would be a time lag between the time at which a syringe 36 or a plunger 32 receives a command to stop moving and the time at which it actually terminates its operation. However, it is possible to terminate the operation of a syringe 36 or a plunger 32 in consideration of such time lag by calculating the descending rate of the fluid level and predicting the time at which a predetermined liquid volume is obtained.

In addition, it is possible to detect the fluid level with the use of an ultrasonic sensor instead of a photosensor. When using an ultrasonic sensor, it is possible to detect the position corresponding to the fluid level by placing an ultrasonic generating element and an ultrasonic detecting element in a nucleic acid isolation column in a manner such that they face each other and detecting whether or not solution is present between the ultrasonic generating element and the ultrasonic detecting element based on the difference between the detection level of the ultrasonic detecting element.

Further, it is possible to detect the fluid level with an image sensor. In such case, the fluid level can be detected by subjecting an image obtained by the image sensor to image processing. Such image processing can be carried out by a control unit 40.

## Claims

1. A method for nucleic acid isolation, wherein nucleic acids of interest are isolated from a liquid sample containing nucleic acids with the use of a nucleic acid isolation column having a nucleic-acid-adsorptive solid phase, comprising the steps of:
allowing nucleic acids to adsorb to the nucleic-acid-adsorptive solid phase by aspirating the liquid sample containing nucleic acids into the nucleic acid isolation column and discharging the liquid sample containing nucleic acids from the nucleic acid isolation column, provided that a certain volume of the liquid sample containing nucleic acids is allowed to remain in the nucleic acid isolation column;
removing nonspecific adsorbing substances from the nucleic-acid-adsorptive solid phase by aspirating a washing reagent into the nucleic acid isolation column and discharging the washing reagent and the nonspecific adsorbing substances from the nucleic acid isolation column, provided that a certain volume of the washing reagent is allowed to remain in the nucleic acid isolation column; and
eluting nucleic acids of interest from the nucleic-acid-adsorptive solid phase by aspirating an elution reagent into the nucleic acid isolation column and discharging the elution reagent and nucleic acids of interest from the nucleic acid isolation column, provided that a certain volume of the elution reagent is allowed to remain in the nucleic acid isolation column.

2. The method for nucleic acid isolation according to claim 1, wherein the washing reagent includes a first washing reagent and a second washing reagent, and wherein the nonspecific adsorbing substances, which cannot be removed from the nucleic-acid-adsorptive solid phase with the use of the first washing reagent, are removed with the use of the second washing reagent.

3. The method for nucleic acid isolation according to claim 1, wherein it is determined whether a certain volume of the liquid sample containing nucleic acids, the washing reagent, or the elution reagent remains in the nucleic acid isolation column by detecting the fluid level in the nucleic acid isolation column with the use of a photosensor.

4. The method for nucleic acid isolation according to claim 1, wherein the liquid sample containing nucleic acids, the washing reagent, or the elution reagent in the nucleic acid isolation column is aspirated into and discharged from the nucleic acid isolation column with the use of a syringe, the pressure in the syringe is detected with the use of a pressure sensor, and it is determined whether a certain volume of the liquid sample containing nucleic acids, the washing reagent, or the elution reagent remains in the nucleic acid isolation column based on the detected pressure value and the distance moved by a plunger of the syringe.

5. The method for nucleic acid isolation according to claim 1, wherein it is determined whether a certain volume of the liquid sample containing nucleic acids, the washing reagent, or the elution reagent remains in the nucleic acid isolation column by detecting the fluid level in the nucleic acid isolation column with the use of an ultrasonic sensor.

6. The method for nucleic acid isolation according to claim 1, wherein it is determined whether a certain volume of the liquid sample containing nucleic acids, the washing reagent, or the elution reagent remains in the nucleic acid isolation column by detecting the fluid level in the nucleic acid isolation column with the use of an image sensor.

7. An instrument for nucleic acid isolation, in which nucleic acids of interest are isolated from a liquid sample containing nucleic acids, comprising:
a nucleic acid isolation column having a nucleic-acid-adsorptive solid phase;
an aspiration and discharge unit for aspirating a solution into the nucleic acid isolation column and discharging the solution therefrom;
a remaining volume detection unit for detecting whether or not a certain volume of the solution remains in the nucleic acid isolation column; and
a control unit for controlling the operation of the aspiration and discharge unit and terminating a discharge operation of the aspiration and discharge unit based on a detection signal from the remaining volume detection unit,
wherein the control unit controls the operation of the aspiration and discharge unit so as to cause elution of nucleic acids of interest from the nucleic-acid-adsorptive solid phase by:
aspirating the liquid sample containing nucleic acids into the nucleic acid isolation column and discharging the liquid sample containing nucleic acids from the nucleic acid isolation column, provided that a certain volume of the liquid sample containing nucleic acids is allowed to remain in the nucleic acid isolation column;
aspirating a washing reagent into the nucleic acid isolation column and discharging the washing reagent and nonspecific adsorbing substances from the nucleic acid isolation column, provided that a certain volume of the washing reagent is allowed to remain in the nucleic acid isolation column; and
aspirating an elution reagent into the nucleic acid isolation column and discharging the elution reagent and nucleic acids of interest from the nucleic acid isolation column, provided that a certain volume of the elution reagent is allowed to remain in the nucleic acid isolation column.

8. The instrument for nucleic acid isolation according to claim 7, wherein the washing reagent includes a first washing reagent and a second washing reagent, and wherein the control unit is used for removing nonspecific adsorbing substances, which cannot be removed from the nucleic-acid-adsorptive solid phase with the use of the first washing reagent, with the use of the second washing reagent.

9. The instrument for nucleic acid isolation according to claim 7, wherein the remaining volume detection unit is a photosensor, and wherein the photosensor detects the fluid level in the nucleic acid isolation column and thereby the control unit determines whether a certain volume of the liquid sample containing nucleic acids, the washing reagent, or the elution reagent remains in the nucleic acid isolation column.

10. The instrument for nucleic acid isolation according to claim 7, wherein the aspiration and discharge unit is a syringe and the remaining volume detection unit is a pressure sensor, and wherein the pressure in the syringe is detected with use of the pressure sensor and thereby the control unit determines whether a certain volume of the liquid sample containing nucleic acids, the washing reagent, or the elution reagent remains in the nucleic acid isolation column based on the detected pressure value and the distance moved by a plunger of the syringe.

11. The instrument for nucleic acid isolation according to claim 7, wherein the remaining volume detection unit is an ultrasonic sensor, and wherein the ultrasonic sensor detects the fluid level in the nucleic acid isolation column and thereby the control unit determines whether a certain volume of the liquid sample containing nucleic acids, the washing reagent, or the elution reagent remains in the nucleic acid isolation column.

12. The instrument for nucleic acid isolation according to claim 7, wherein the remaining volume detection unit is an image sensor, and wherein the image sensor detects the fluid level in the nucleic acid isolation column and thereby the control unit determines whether a certain volume of the liquid sample containing nucleic acids, the washing reagent, or the elution reagent remains in the nucleic acid isolation column.


SEQUENCE LISTING
<110>  Hitachi High-Technologies Corporation

<120>  INSTRUMENT AND METHOD FOR NUCLEIC ACID ISOLATION

<130>  EPA-45140

<150>  JP 2007-176325
<151>  2007-07-04

<160>  6

<170>  PatentIn version 3.4

<210>  1
<211>  27
<212>  DNA
<213>  Artificial

<220>
<223>  primer

<400>  1
gcggatccga gttactctcg tttttgc                                          27

<210>  2
<211>  29
<212>  DNA
<213>  Artificial

<220>
<223>  primer

<400>  2
gcaagctttc taacaacagt agtttccgg                                        29

<210>  3
<211>  46
<212>  DNA
<213>  Artificial

<220>
<223> primer

<400> 3
cctgctgcta tgcctcatct tctttgacaa acgggcaaca tacctt 46

<210> 4
<211> 44
<212> DNA
<213> Artificial

<220>
<223> primer

<400> 4
gataaaacgc cgcagacaca tccttccaac ctcttgtcct ccaa 44

<210> 5
<211> 19
<212> DNA
<213> Artificial

<220>
<223> primer

<400> 5
ggtggttgat gttcctgga 19

<210> 6
<211> 20
<212> DNA
<213> Artificial

<220>
<223> primer

<400> 6
caaaattcgc agtccccaac 20